Europäisches Patentamt

European Patent Office

Office européen des brevets

(11) Numéro de publication: **0 288 388 B1**

## FASCICULE DE BREVET EUROPEEN

(45) Date de publication de fascicule du brevet: **13.04.94**   (51) Int. Cl.⁵: **G01N  33/577**, G01N 33/569

(21) Numéro de dépôt: **88400977.0**

(22) Date de dépôt: **21.04.88**

(54) **Immunodiagnostic des parasitoses dues à un protozoaire chez les mollusques.**

(30) Priorité: **24.04.87 FR 8705856**

(43) Date de publication de la demande:
**26.10.88 Bulletin  88/43**

(45) Mention de la délivrance du brevet:
**13.04.94 Bulletin  94/15**

(84) Etats contractants désignés:
**ES FR GB IT**

(56) Documents cités:
**WO-A-83/00929**
**WO-A-83/01837**
**WO-A-86/02098**
**FR-A- 2 543 970**

**BIOLOGICAL ABSTRACTS, vol. 77, 1984, Biological Abstracts Inc.; M. COMPS, no.
76034&NUM;**

**BIOLOGICAL ABSTRACTS, vol. 83, 1987, Biological Abstracts Inc.; R.A. ELSTON et al., no.
95635&NUM;**

**BIOLOGICAL ABSTRACTS, vol. 84, 1987, Biological Abstracts Inc.; R.A. ELSTON et al., no.
96907&NUM;**

(73) Titulaire: **SANOFI S.A.**
**40, Avenue George V**
**F-75008 Paris(FR)**

Titulaire: **INSTITUT FRANCAIS DE RECHER-
CHE POUR L'EXPLOITATION DE LA MER
(IFREMER)**
**66 avenue d'Iéna**
**F-75116 Paris(FR)**

(72) Inventeur: **Grizel, Henri**
**Rue de l'Atlantique**
**Dirée, F-17530 Arvert(FR)**
Inventeur: **Mialhe, Eric**
**14, rue Maurice Brunet**
**F-17390 La Tremblade(FR)**
Inventeur: **Paolucci, Francis**
**145, Impasse de la Voie Romaine**
**F-34090 Montpellier(FR)**
Inventeur: **Rogier, Hervé**
**63C, Résidence de May, 790, rue du Fesquet**
**F-34100 Montpellier(FR)**

(74) Mandataire: **Gillard, Marie-Louise et al**
**Cabinet Beau de Loménie**
**158, rue de l'Université**
**F-75340 Paris Cédex 07 (FR)**

BIOLOGICAL ABSTRACTS, vol. 83, 1987, Biological Abstracts Inc.; S. BOUGRIER et al., no. 52971&NUM;

BIOLOGICAL ABSTRACTS, vol. 75, 1983, Biological Abstracts Inc.; M. BREHELIN et al., no. 80873&NUM;

AOUAT. LIVING RESOUR., vol. 1, 1988; E. MIALKE, pp. 67-69&NUM;

C. R. Acad. SCI., vol. 296, 1983, Paris (FR); M. COMPS, serie III, pp. 931-934&NUM;

# EP 0 288 388 B1

**Description**

La présente invention concerne des anticorps monoclonaux dirigés spécifiquement contre un antigène d'un protozoaire parasitant un mollusque. Elle concerne plus particulièrement parmi ces anticorps monoclonaux ceux qui sont dirigés contre un antigène d'un protozoaire appartenant au phylum des Ascetospora tel que défini par LEVINE N,D. et al.((1980) A newly revised classification of the Protozoa., 27, 37 - 58). Et parmi ces anticorps elle concerne plus spécialement ceux qui sont dirigés contre un antigène du protozoaire Bonamia ostreae parasite de l'espèce d'huître plate Ostrea edulis L.

Il est connu que les mollusques, notamment les mollusques marins, peuvent être parasités par un protozoaire. Plusieurs protozoaires pathogènes ont pu être identifiés,notamment parmi les genres Minchinia. Haplosporidium, et Marteilia (GRIZEL H. et al. (1974) Science et Pêches, Bull. Inst. Pêches Marit., No. 240, 7 - 30 ; PERKINS F.O. (1979) Mar. Fish. Rev., 41 (1 - 2), 25 - 37). Un autre protozoaire Bonamia ostreae à l'origine d'une parasitose des parcs d'élevage d'huîtres plates français a fait l'objet, depuis sa mise en évidence (COMPS M. et al. (1980) C.R. Acad. Sc. Paris, 290, sér. D, 383-384), de nombreux travaux portant tant sur sa structure (PICHOT Y. et al. (1980) Rev. Trav. Inst. Pêches Marit., 43 (1), 131 - 140) que sur la situation épidémiologique consécutive à sa présence (TIGE G. et al. (1981) Science et Pêche, Bull. Inst. Pêches Marit., No. 315, 13 - 20).

Ces parasitoses déciment les populations de mollusques. Elles peuvent conduire à la disparition d'espèces. Elles ont bien entendu, lorsqu'elles concernent des mollusques d'élevage, un impact d'ordre économique important (GRIZEL H. (1983) Cons. Int. Explor. Mer, Gen. : 9, 30 p), surtout lorsqu'elles prennent la forme d'une épizootie endémique. C'est précisément le cas de la parasitose de l'huître Ostrea edulis L. dont l'agent étiologique est Bonamia ostreae, pour lequel il a été constaté expérimentalement qu'un seul individu pouvait provoquer la mort d'une huître à laquelle il avait été inoculé quatre mois plus tôt. Cette parasitose, après avoir été identifiée successivement dans différents parcs à huîtres français, l'est maintenant dans certains bassins ostréicoles de plusieurs Etats européens, notamment l'Espagne, l'Irlande, les Pays-Bas et le Royaume-Uni, et des Etats-Unis d'Amérique; il peut également être noté que l'on suspecte à présent un protozoaire du genre Bonamia d'être à l'origine, en Nouvelle-Zélande, d'une parasitose de l'espèce d'huître plate Ostrea luteria.

L'importance des enjeux économiques liés à ces parasitoses a conduit à rechercher des mesures permettant sinon de les éradiquer du moins de les enrayer.

Ces mesures sont le plus souvent d'ordre prophylaxique, lorsqu'il n'a pas été trouvé pour le mollusque concerné une race résistante à la parasitose ou lorsque aucun traitement antiparasitaire ne s'est avéré efficace. Elles consistent alors essentiellement à empêcher l'introduction dans les parcs d'élevage sains d'animaux déjà parasités. Elles nécessitent donc pour leur mise en oeuvre que l'on dispose d'une méthode de diagnostic performante se caractérisant par sa commodité d'exécution et sa fiabilité.

Une telle méthode de diagnostic n'a pas été décrite. En effet, la seule méthode connue consiste à pratiquer des examens histologiques sur les mollusques. Cette méthode est peu adaptée à une pratique courante en raison des moyens requis pour sa mise en oeuvre et du recours nécessaire à un spécialiste pour l'interprétation des résultats. Elle ne permet pas d'autre part, à proprement parler,de quantifier le degré d'infestation des mollusques parasités. Un autre inconvénient important est le délai séparant la préparation des échantillons et la disponibilité des résultats.

La demanderesse a maintenant trouvé une méthode simple d'emploi et fiable se fondant sur l'utilisation d'anticoprs monoclonaux. Il lui est, en effet, apparu, étant donné que les mollusques sont incapables, face à une infestation parasitaire, de développer une réponse immunitaire à médiation humorale de type anticorps, qu'il fallait nécessairement mettre en évidence les protozoaires eux-mêmes. Or un anticorps monoclonal reconnaissant spécifiquement un antigène d'un protozoaire constitue un moyen sûr pour sa mise en évidence.

L'invention concerne selon un premier aspect, un procédé de détetion d'un protozoaire du genre Bonamia parasitant les cellules sanguines d'un mollusque qui consiste à prélever un échantillon d'hémolymphe d'un mollusque parasité par ledit protozoaire, ledit échantillon étant ensuite soumis à un traitement favorisant l'éclatement des cellules sanguines, et à détecter la présence dudit protozoaire par une technique immunologique à l'aide d'anticorps monoclonaux reconnaissant spécifiquement ledit protozoaire.

L'obtention d'anticorps monoclonaux reconnaissant un antigène d'un protozoaire a déjà été décrite dans le cadre de travaux portant sur des protozoaires du genre Leishmania, qui, parasites de globules blancs humains, sont d'un point de vue phyllogénétique sensiblement éloignés des protozoaires parasitant les mollusques. Mais les publications disponibles (notamment la demande de brevet internationale WO 86/02098) ne permettent pas de surmonter les difficultés particulières qui, liées à l'isolement et à la purification d'un protozoaire à partir de mollusques qu'il a parasités, étaient inexistantes pour les protozoaires du genre Leishmania qu'il est possible de cultiver et pour lesquels il existe des souches pures dans diverses collections.

La demanderesse a précisément mis au point un procédé d'obtention d'anticorps monoclonaux reconnaissant un antigène d'un protozoaire parasitant un mollosque en concevant un mode de purification dudit protozoaire, permettant la préparation d'une suspension purifiée et concentrée indispensable pour une immunisation correcte d'animaux de laboratoire.

Pour la mise en oeuvre de l'invention selon ses différents aspects, par mollusque il faut entendre tout animal appartenant à l'embranchement des mollusques qui comprend lui-même trois classes principales : les gastéropodes, les lamellibranches et les céphalopodes. L'invention peut concerner tout mollusque quelle que soit l'influence de l'homme sur son environnement : il peut s'agir ou non d'un mollusque d'élevage. Il suffit que ledit mollusque soit parasité par un protozoaire. L'invention convient tout particulièrement à l'obtention d'anticorps monoclonaux pour la détection et la mesure de leur degré d'infestation par des protozoaires du genre Bonamia et plus particulièrement par ceux appartenant à l'espèce Bonamia Ostreae, qui ont pour cible les cellules sanguines du mollusque.

L'invention concerne selon un autre aspect tout anticorps monoclonal reconnaissant de manière spécifique un antigène d'un protozoaire parasitant un mollusque.

Elle concerne plus particulièrement tout anticorps monoclonal reconnaissant de manière spécifique un antigène de Bonamia ostreae. D'une manière préférée, elle concerne l'anticorps monoclonal sécrété par l'hybridome dont un échantillon d'une culture a été déposé auprès de l'"European Collection of Animal Cell Cultures" (ECACC, Royaume-Uni) le 23 avril 1987 et auquel ladite collection a attribué la référence 87042307. Cet anticorps désigné ci-après sous la référence 2OB2-IBI2 appartient à la classe IgG2a et a un point isoélectrique de 6,9 à 7,3. Il présente relativement à neuf autres anticorps monoclonaux sélectionnés et produits par la demanderesse d'autres caractéristiques qui seront présentées ci-après dans l'exemple 4.

L'invention concerne selon un autre aspect un procédé d'obtention desdits anticorps monoclonaux. Ce procédé comporte les étapes successives suivantes :

- l'extraction dudit protozoaire à partir de l'hémolymphe d'un mollusque parasité par broyage du corps du mollusque duquel on a ôté tout organe de nature fibreuse en présence d'un liquide d'homogénéisation à base d'eau de mer additionnée de 0,5 à 2% (v/v) d'un tensioactif, clarification du broyat et concentration des éléments particulaires dudit broyat, séparation des cellules du protozoaire,
- la préparation d'une suspension purifiée du protozoaire ainsi extrait, et
- l'utilisation de ladite suspension pour obtenir les anticorps monoclonaux spécifiques dudit protozoaire.

La préparation d'une suspension purifiée du protozoaire fait intervenir une opération de broyage du corps des mollusques menée de telle manière que les cellules de mollusque contenant les protozoaires éclatent et que les protozoaires sont recueillis intacts après plusieurs opérations de centrifugation permettant progressivement l'élimination des cellules de mollusque et des débris cellulaires de toutes sortes contenus initialement dans le broyat.

De manière à recueillir le plus de protozoaires possible, il est avantageux de soumettre au broyage le corps entier des mollusques ; ce mode opératoire met à profit le rôle prépondérant que peuvent jouer, pour l'infestation d'un mollusque, ses cellules sanguines qui, le système circulatoire des mollusques n'étant pas clos, se trouvent dispersées dans l'ensemble des tissus. Cependant il convient d'en ôter au préalable tout organe de nature fibreuse tel que chez les mollusques de la classe des lamellibranches le muscle adducteur de la coquille bivalve. En effet, un organe de cette nature conduirait à la formation d'agrégats et ferait obstacle à une homogénéisation correcte du broyat.

L'obtention d'un broyat, dans lequel les protozoaires sont préservés, implique l'utilisation d'un liquide, qualifié ci-après de liquide d'homogénéisation, maintenant autour des protozoaires un environnement protecteur. Ce liquide doit être ajouté dès la phase de broyage. De manière avantageuse on utilise de l'eau de mer dépourvue de micro-organismes contaminants notamment bactériens, dont la pression osmotique a été ajustée - si elle avait une valeur inférieure - à environ 1 osmole à l'aide de chlorure de sodium et additionnée d'un tensioactif. D'une manière préférée un tensioactif neutre est utilisé. Du monooleate de polyoxyéthylènesorbitane 20 du type de celui commercialisé sous l'appellation Tween® 80 est particulièrement adapté. Le tensioactif est ajouté à l'eau de mer à raison de 0,5 à 2% (v/v) et d'une manière préférée à raison de 1% (v/v).

Un appareillage particulièrement bien adapté au broyage des mollusques est celui commercialisé sous la dénomination Ultra-Turrax par la Société IKA JAUKE und KUNKEL (République Fédérale Allemande). Parmi les axes générateurs dont ce matériel peut être équipé, celui portant la référence No. 18K est préféré.

Le broyage est avantageusement effectué en deux étapes : une homogénéisation succédant à une première phase de lacération du corps des mollusques.

Le broyat homogénéisé est ensuite soumis à un traitement de clarification et de concentration des éléments particulaires. Ce traitement comprend avantageusement une filtration suivie d'une centrifugation de la suspension des éléments particulaires. La filtration est destinée à permettre l'élimination des plus gros éléments particulaires. La centrifugation est destinée à permettre l'élimination des solutés et la concentration des éléments particulaires restants, en reprenant le culot de centrifugation à l'aide de liquide d'homogénéisation sous un volume inférieur au volume initial.

Par exemple, la filtration est réalisée sur successivement trois toiles de nylon de maille égale à 300, 60 puis 25 $\mu$m et la centrifugation du filtrat est conduite à 2 000 g pendant 30 minutes.

Ce premier traitement doit être suivi d'un traitement d'élimination des éléments particulaires autres que les protozoaires recherchés. Ce traitement comprend avantageusement plusieurs centrifugations différentielles, par exemple sur saccharose, en gradient ou non, se succédant de manière à éliminer progressivement selon leur taille et leur densité les éléments particulaires indésirables.

Une ultime centrifugation de nature isopycnique permet, si nécessaire, d'atteindre un haut degré de purification. L'ensemble de ces purifications est réalisé selon les techniques connues de l'homme du métier. Il est possible de récueillir en définitive jusqu'à $10^6$ à $10^7$ protozoaires à partir d'un mollusque parasité.

La suspension purifiée de protozoaires obtenue est mise en oeuvre pour immuniser des animaux de laboratoire. Des souris peuvent être commodément utilisées. Le schéma et le protocole d'immunisation sont établis, en tenant compte de la quantité de protozoaires recueillis, selon les critères habituels de l'homme du métier.

L'hybridation lymphocytaire est réalisée à partir des lymphocytes de la rate d'une souris immunisée et de cellules myélomateuses selon la technique de base décrite par KOHLER G. et MILSTEIN C. ((1975) Nature, 256, 495-497).

La sélection des hybridomes sécrétant un anticorps monoclonal reconnaissant spécifiquement un antigène du protozoaire concerné. fait appel à la détection des anticorps sécrétés par chaque hybridome, par exemple à l'aide d'un test radio-immunologique. Les cellules sont ensuite clonées, par exemple selon la technique de dilution limite. L'activité anticorps de chaque clone est contrôlée. L'homme de l'art est en mesure de déterminer parmi les techniques connues un choix adapté à chaque cas particulier.

Les hybridomes sélectionés peuvent ensuite être congelés dans l'azote liquide. Une fois remis en culture sur plaques de culture, ils sécrètent l'anticorps que l'on peut caractériser dans les surnageants de culture. Il est également possible de préparer les anticorps à partir d'un liquide d'ascite lorsque l'on désire obtenir une concentration élevée d'anticorps, Ces différents modes de production sont bien connus de l'homme du métier.

Les anticorps monoclonaux collectés peuvent bien entendu être ensuite purifiés, selon l'emploi auquel on les destine, en mettant en oeuvre une technique connue. Une technique peut avantageusement être choisiepour une utilisation des anticorps monoclonaux selon l'invention en vue du diagnostic in vitro de parasitoses. Elle consiste à soumettre la préparation d'anticorps monoclonaux à une chromatographie d'affinité en utilisant une colonne de protéine A insolubilisée.

Les anticorps monoclonaux selon l'invention constituent des outils de diagnostic d'un grand intérêt grâce à la haute spécificité qui caractérise la liaison pouvant s'établir entre un anticorps et un antigène. Ils peuvent être utilisés selon diverses techniques connues en soi de l'homme du métier. Mis en oeuvre de préférence sous forme purifiée, ils peuvent être fixés par adsorption sur un support insoluble tel que par exemple une plaque de microtitration. Ils peuvent également être couplés à un marqueur. Toutes sortes de marqueurs connus conviennent : il peut s'agir notamment d'un marqueur fluorescent, d'un isotope radioactif ou encore d'une enzyme. De manière avantageuse, les anticorps monoclonaux selon l'invention, par exemple adsorbés sur un support solide ou marqués, mis à la disposition des utilisateurs, constituent l'un des composants d'un kit de diagnostic comportant les éléments essentiels à la réalisation de la détection ou du dosage envisagé.

L'invention concerne précisément selon un autre aspect l'utilisation desdits anticorps monoclonaux en tant qu'outils de diagnostic in vitro des parasitoses affectant les mollusques dont l'agent étiologique est un protozoaire. Ces anticorps monoclonaux permettent, au niveau de chaque animal testé, la détection des protozoaires parasites et par un dosage la mesure de leur degré d'infestation. Elle concerne donc aussi un kit de diagnostic comportant un échantillon d'au moins une préparation à base d'un anticorps monoclonal selon l'invention.

L'invention apporte ainsi une solution fiable à l'important problème que constitue le suivi de l'état sanitaire des mollusques qui seul permet la mise en place d'une stratégie de prophylaxie zoosanitaire.

L'invention concerne selon un autre aspect les hybridomes sécréteurs d'un anticorps monoclonal reconnaissant de manière spécifique un antigène d'un protozoaire parasitant un mollusque. Un hybridome préféré est celui qui a été déposé à l'ECACC sous la référence 87042307.

Dans ce qui suit des exemples de réalisation et de mise en oeuvre des différents aspects de l'invention sont présentés. Bien qu'ils se rapportent à des anticorps monoclonaux dirigés contre le protozoaire Bonamia ostreae il doit être compris que l'invention ne se limite pas à ces seuls anticorps et en particulier à leur obtention et à leur utilisation et concerne d'une manière générale les anticorps monoclonaux reconnaissant un antigène d'un protozoaire parasitant un mollusque et plus particulièrement ceux reconnaissant un antigène d'un protozoaire appartenant au phyllum des Acetospora.

Différents tampons, cités ci-après dans les exemples, seront désignés par commodité par une abréviation. Il s'agit des tampons :

- PBS $KH_2PO_4$ 10 mmol/l, NaCl 150 mmol/l, pH ajusté à 7,4
- PBS1 Tampon PBS additionné, à raison de 0,1% (p/v), de sérum-albumine bovine commercialisée par la Société SIGMA sous la référence A 4503.
- PBS2 Tampon PBS additionné, à raison de 0,5% (p/v), de sérum-albumine bovine (SIGMA).
- PBS3 Tampon PBS2 additionné, à raison de 0,5% (v/v), de monolaurate de polyoxyéthylènesorbitane 20 commercialisé par la Société MERCK (E.U.A.) sous la référence 822 184 et sous l'appellation Tween® 20, ci-après désigné polysorbate 20.
- PBS4 Tampon PBS additionné, à raison de 2% (p/v), de sérum-albumine bovine (SIGMA).
- PBS5 Tampon PBS4 additionné, à raison de 0,75% (p/v), de glycine commercialisée par la Société MERCK (E.U.A.) sous la référence 4201.
- PBS6 Tampon PBS additionné, à raison de 0,5 (v/v) de glutaraldéhyde commercialisé par la Société SIGMA sous la référence G 6257.
- IF Tampon pour immunofluorescence commercialisé par la Société DiagnosticsPasteur (FRANCE) sous la référence 74903.
- IF-EVANS Tampon IF additionné, à raison de 1/10000 (v/v), de bleu d'EVANS
- TBS Tri(hydroxyméthyl)aminométhane : 10 mmol/l, NaCl 100 mmol/l, pH ajusté à 7,4.
- TBS1 Tampon TBS additonné, à raison de 1% (v/v), de polysorbate 20
- TBS2 Tampon TBS additonné, à raison de 0,1% (v/v), de polysorbate 20.

Des plaques de microtitration sont utilisées. Il s'agit de plaques en polystyrène commercialisées par la Société NUNC (E.U.A.) sous la référence 2 - 69620.

Les plaques de culture mises en oeuvre sont celles commercialisées par la Société FALCON (E.U.A.) sous la référence 3072.

Des filtres de nitrocellulose de 6 mm de diamètre sont placés dans les puits des plaques de microtitration. Ils sont découpés à l'emporte-pièce à partir de filtres de nitrocellulose commercialisés par la Société SCHLEICHER et SCHULL (R.F.A.) sous les références BA 83/No. 401380.

EXEMPLE

1. PREPARATION D'UNE SUSPENSION DE Bonamia Ostreae

a) Broyage

10 huîtres provenant d'un parc à huîtres du bassin de la baie de Quiberon (Morbihan - FRANCE) infectées par Bonamia ostreae sont préparées.

Les coquilles sont détachées des corps et le muscle adducteur est découpé et éliminé.

Les 10 corps ainsi préparés sont introduits dans un bécher d'une contenance de 500 ml et dont la section présente un diamètre de 10 cm. On ajoute 300 ml d'eau de mer dont la pression osmotique a été ajustée à 1 osmole, filtrée sur une membrane présentant des pores ayant au maximum un diamètre de 0,22 µm et additionnée, à raison de 1% (v/v), de monolaurate de polyoxyéthylènesorbitane 20 commercialisé par la Société SIGMA (E.U.A.) sous l'appellation Tween® 80 et sous la référence p 1754. Ce liquide d'homogénéisation est désigné, par commodité, ci-après dans la suite du texte, par l'abréviation E.M.F.T..

L'axe générateur No. 18 K monté sur un matériel ULTRA-TURRAX est introduit dans le bécher puis est mis en rotation à environ 8 000 tours par minute. Quand il n'y a plus de masse tissulaire observable, la rotation est arrêtée et l'axe générateur est déplacé hors du bécher.

Le broyat obtenu est transféré dans une série de cylindres de Potter de Thomas dont le diamètre au niveau du tube proprement dit est légèrement supérieur à celui (25 mm) de l'axe générateur.

L'axe générateur en rotation à 8 000 tours par minute est plongé dix fois à la suite l'une de l'autre dans le broyat de l'un des cylindres. La même opération est répétée sur chacun de ces cylindres. Les contenus des différents cylindres sont regroupés. Il constituent un broyat homogénéisé que l'on filtre sur successivement trois toiles de nylon de maille égale à 300, 60 puis 25 μm.

Ce broyat homogénéisé et filtré contient des cellules d'huîtres intactes, des débris de telles cellules, notamment des noyaux, certains des organismes contaminants et les protozoaires recherchés.

b) Centrifugations

Le broyat obtenu en a est réparti dans 10 tubes. Chaque tube est soumis à une centrifugation à 2 000 g pendant 30 minutes à 8°C. Le culot C1 obtenu est remis en suspension par homogénéisation à l'ULTRA-TURRAX dans 10 ml d'E.M.F.T. (suspension S1).

Chaque suspension S1 est alors soumise à une centrifugation différentielle. 10 ml de la suspension S1 sont déposés à la surface de 30 ml d'une solution à 20% (p/p) de saccharose dans de l'E.M.F.T.; après centrifugation à 2 000 g pendant 30 minutes à 8°C, le culot C2 obtenu est remis en suspension dans 5 ml d'E.M.F.T. (suspension S2). Cette centrifugation différentielle a permis l'élimination de débris de petite taille et des micro-organismes contaminants. Chaque suspension S2, sous un volume de 5 ml, est déposée sur un gradient de densité discontinu de saccharose préparé à partir de 15 ml d'une solution à 20% (p/p) de saccharose dans de l'E.M.F.T. et de 30 ml d'une solution à 40% (p/p) de saccharose dans de l'E.M.F.T. Après une centrifugation à 2 000 g pendant 30 minutes à 8°C, on constate que les protozoaires recherchés se sont répartis à l'interface des deux solutions. Les débris cellulaires plus grands ou plus denses ont pénétré dans la fraction correspondant à une concentration en saccharose de 40%. Les fractions correspondant à l'interface sont recueillis et rassemblées.

Les suspensions des protozoaires recherchées ainsi obtenues issues des 10 suspensions S2 sont regroupées, diluées volume à volume avec de l'E.M.F.T. et réparties dans 3 pots à centrifugation à 2 000 g pendant 30 minutes à 8°C. Les 3 culots C3 sont repris dans de l'E.M.F.T.. La suspension S3 obtenue contient sous un volume de 5 ml l'ensemble des protozoaires recherchés issus des 10 huîtres traitées. Cette suspension peut être utilisée telle quelle. La poursuite de la purification par une centrifugation isopycnique permet l'obtention de résultats d'une qualité accrue.

Cette dernière étape de purification est conduite en déposant la suspension S3, préalablement répartie dans 10 tubes, sur un double gradient de densité et de tonicité discontinu d'un colloïde polydispersé de silice couverte de polyvinyl pyrrolidone commercialisé sous la dénomination PERCOLL® par la Société PHARMACIA (Suède). Partant d'une solution mère de PERCOLL dont la pression osmotique a été ajustée au préalable à 1 400 mosm à l'aide de NaCl ajouté jusqu'à ce que la concentration en NaCl atteigne 0,7 mol/l, cinq solutions de PERCOLL, à 30%, 40%, 50%, 60%, et 70% (v/v) dans de l'E.M.F.T., sont préparées. Le gradient de pression osmotique créé ainsi est compris entre 1 100 mosm (correspondant à une concentration de PERCOLL de 30%) et 1 300 mosm (correspondant à une concentration de PERCOLL de 70%).

Après centrifugation à 2 000 g pendant 30 minutes à 8°C, les protozoaires recherchés se concentrent principalement à l'interface 60-70%. Les fractions correspondant à cette interface sont recueillies et constituent la suspension SP1. L'interface 50-60% contient également des protozoaires mais ceux-ci sont en quantité moindre et associés à quelques débris.

La suspension SP1 ne contient que des protozoaires, auxquels peuvent néanmoins rester accrochés des fragments de membranes de la vacuole parasitophore de la cellule d'huître. Des examens ultrastructuraux et des infections expérimentales ont confirmé qu'ils sont infectieux et que leur structure n'a pas été altérée.

Le pool des 10 suspensions SP1 issues du traitement des 10 huîtres parasitées, recueilli sous un volume de 6 ml, contient environ $5.10^7$ protozoaires. Ce pool peut être utilisé tel quel. Cependant, pour une application telle que l'adsorption des protozoaires à la surface d'une plaque de microtitration, il est nécessaire d'en éliminer le PERCOLL. Ceci peut être obtenu en centrifugeant, à 2 000 g pendant 30 minutes à 8°C, 12 ml de la suspension obtenue par dilution volume à volume de suspension SP1 dans de l'E.M.F.T., posés sur un coussin de 2 ml d'une solution à 20% (p/p) de saccharose dans de l'E.M.F.T.. Le culot repris dans 20 ml d'E.M.F.T. constitue la suspension SP2 qui est dépourvue de PERCOLL et contient environ $2,5.10^6$ protozoaires par ml.

## 2. PREPARATION D'UNE SUSPENSION PURIFIEE DE HAPLOSPORIDIUM ARMORICANUM

Une suspension purifiée de spores de Haplosporidium armoricanum a été obtenue en soumettant des huîtres de l'espèce Ostrea edulis L. parasitées prélevées dans un gisement naturel de la région de Thau (Hérault - FRANCE) au protocole mis en oeuvre à l'exemple 1. Ces spores présentant une densité légèrement supérieure à celle des cellules de Bonamia ostreae se concentrent lors de la centrifugation isopycnique non pas à l'interface 60-70% mais dans le fond de la fraction 70%.

7

### 3. OBTENTION D'ANTICORPS MONOCLONAUX ANTI-BONAMIA OSTREAE

#### 3.1. Hybridation lymphocytaire

a) Immunisation de souris de laboratoire :

6 souris femelles de la lignée Balb/c reçoivent chacune, au temps zéro, 800 $\mu$l de la suspension SP1 obtenue à l'exemple 1 diluée au demi dans de l'adjuvant de Freund, à raison de 500 $\mu$l par voie intrapéritonéale, 200 $\mu$l par voie intraveineuse et 100 $\mu$l par voie intramusculaire. Chaque souris est ensuite soumise à sept rappels réalisés chacun en suivant ce protocole. Le premier de ces rappels est effectué un mois après la série initiale d'injections et les six rappels suivants se succèdent de mois en mois. Le septième rappel est suivi un mois plus tard d'un dernier rappel effectué en présence de prométhazine (Sigma - référence p 4651). Les souris sont sacrifiées trois jours après le dernier rappel.

b) Fusion :

$300.10^6$ lymphocytes B recueillis à partir de la rate d'une souris immunisée sont mis au contact de $150.10^6$ cellules de la lignée myélomateuse P3 x 63 Ag 8-653, en présence de polyéthylèneglycol commercialisé sous la référence PEG 1540 par la Société RIEDEL-DE-HAEN (R.F.A.). La fusion est réalisée selon la technique décrite par J. GODING ((1980) J. Immunol. Methods, 39, 285). La suspension cellulaire est distribuée, à raison de cellules par puits, dans les puits de plaques de culture contenant chacun $10^4$ macrophages péritonéaux de souris. Du milieu HAT, contenant en mélange de l'hypoxanthine, de l'aminoptérine et la thymidine, est ajouté 25 h après la fusion.

#### 3.2. Sélection des hybridomes sécrétant un anticorps monoclonal reconnaissant de manière spécifique un antigène de Bonamia ostreae :

Un mode opératoire, se fondant sur la détection par une méthode radio-immunologique indirecte d'une activité anticorps des surnageants de culture des puits des plaques de culture vis-à-vis d'une part d'une suspension purifiée de Bonamia ostreae et d'autre part d'un broyat préparé à partir d'une huître plate de l'espèce Ostrea edulis L. exempte de Bonamia ostreae, est mis en oeuvre.

#### 1 - Préparation de plaques de microtitration

a) plaques portant des protozoaires adsorbés en surface :

Chaque puits reçoit 50 $\mu$l d'une solution de poly-L-lysine à 20 $\mu$g/l dans du tampon PBS.

Après un contact de 30 minutes à 20°C, les plaques sont vidées par retournement et chaque puits reçoit 100 $\mu$l de la suspension SP2 de Bonamia ostreae obtenue à l'exemple 1, soit environ 250 000 protozoaires. Les plaques sont centifugées à 2 000 g pendant 30 minutes à 8°C. Puis 20 $\mu$l de tampon PBS6 sont versés dans chaque puits. Les plaques sont alors incubées 8 minutes à 20°C et lavées deux fois avec le tampon PBS. Les puits sont ensuite saturés avec 200 $\mu$l de tampon PBS5. Après un contact de 30 minutes à 20°C, les puits sont lavés avec le tampon PBS. Enfin, les puits ayant reçu 100 $\mu$l de tampon PBS4, les plaques sont congelées à - 20°C.

b) Plaques témoins :

Elles sont préparées comme indiqué ci-dessus mais la suspension SP2 est remplacée par le broyat d'une huître exempte de Bonamia ostreae. Des huîtres non parasitées originaires du gisement de Belle-Isle - (Morbihan FRANCE) exempt de Bonamia ostreae ont été maintenues en élevage en laboratoire pendant 12 mois et à l'issue de cette période un contrôle histologique a été effectué afin de vérifier l'absence de Bonamia ostreae. Pour la préparation du broyat d'essai : une huître est broyée dans 500 ml d'eau de mer et le broyat est filtré sur une toile de nylon ayant une maille de 25 $\mu$m.

#### 2 - Test de détection

Au moment de l'utilisation, les plaques de microtitration sont décongelées et lavées trois fois avec le tampon PBS. Chaque puits reçoit 50 $\mu$l de l'un des surnageants de culture obtenus en 1.2.B et 50 $\mu$l de tampon PBS3. Après une incubation de 2 heures à 37°C, les plaques sont lavées trois fois avec une solution de lavage constituée d'une solution de NaCl 150 mmol/l additionnée de polysorbate 20 à raison de 0,5% (v/v). Un anticorps de mouton anti-immunoglobulines de souris marqué à l'iode 125, commercialisé par la Société ZYMED (E.U.A.), est dilué avec le tampon PBS3 de manière à présenter une activité de $18.10^6$ Bq/100 $\mu$l. Il est ensuite réparti à raison de 100 $\mu$l par puits. Après une incubation d'une heure à 37°C, les plaques sont lavées cinq fois avec la solution de lavage et la radioactivité de chaque puits est mesurée.

3 - Résultats

La mesure de l'activité des surnageants de culture mesurée dans les puits des plaques de microtitration permet de repérer ceux des puits de plaques de culture contenant au moins un hybridome sécrétant un anticorps monoclonal :
- spécifiquement anti-Bonamia ostreae (activité anticorps de type BO),
- ou anti-huître non parasité par Bonamia ostreae (activité anticorps de type HS),
- ou anti-Bonamia ostreae et anti-huître non parasitée par Bonamia ostreae (activité anticorps de type NS).

Le tableau 1 présente à titre d'exemple des mesures effectuées à partir de trois surnageants de culture présentant une activité anticorps de type BO, HS ou NS. ; la fixation non specifique de l'anticorps de mouton anti-immunoglobulines de souris marqué à l'iode 125 à été mesurée sur des puits préparés comme indiqué ci-dessus au paragraphe 2 en y déposant toutefois non 50 $\mu$l d'un surnageant de culture mais 50 $\mu$l de milieu de culture neuf (milieu RPMI 1640 commercialisé par la Société GIBCO (E.U.A.) additionné à raison de 10% (v/v) de sérum de veau foetal).

TABLEAU 1

| | | Activité(cpm) mesurée dans les puits des plaques de microtitration | |
| --- | --- | --- | --- |
| | | Plaques de microtitration avec protozoaires adsorbés en surface | plaques de microtitration témoin |
| type de l'activité | BO | 2 000 | 500 |
| anticorps | HS | 100 | 10 000 |
| du surnageant | NS | 4 000 | 8 000 |
| ajouté aux puits | | | |
| Fixation non spécifique | | 50 | 400 |

En définitive sur les I 718 puits des plaques de culture testés, 703 contenaient au moins un hybridome entrant dans l'une des trois catégories.

3.3. Clonage des hybridomes et production des anticorps

8 puits des plaques de culture ont été clonés par la technique de dilution limite. 10 clones d'hybridomes sécrétant un anticorps monoclonal spécifiquement anti-Bonamia ostreae ont été sélectionnés. Ces clones ont été congelés dans l'azote liquide. Les anticorps qu'ils sécrètent ont été purifiés, à partir de liquide d'ascite provenant de souris Balb/c femelles, par chromatographie d'affinité sur un gel de Sepharose ® protéine A commercialisé par la Société PHARMACIA (Suède) selon la technique décrite par EY P.L. et al. (1978) Immunochemistry, 15, 429-436).

4. CARACTERISATION DES ANTICORPS MONOCLONAUX SPECIFIQUEMENT ANTI-BONAMIA OSTREAE

4.1. Caractérisation immunologique

1) Mode opératoire :

La caractérisation immunologique des différents épitopes reconnus par 10 anticorps monoclonaux sélectionnés a été effectuée par une compétition, entre l'un des anticorps marqué de manière classique à l'iode 125 et un autre de ces anticorps non marqué, pour leur fixation sur les protozoaires adsorbés sur un filtre de nitrocellulose.

Un filtre de nitrocellulose de 6 mm de diamètre est introduit dans chacun des puits d'une plaque de microtitration.

25 μl de la suspension SP1 obtenue à l'exemple 1, ou d'un broyat d'huître exempte de Bonamia ostreae sont introduits dans chaque puits. Après une incubation de 18 heures à 37°C, chaque puits est saturé avec 200 μl d'un tampon PBS2 pendant 2heures à 37°C. Les puits sont ensuite lavés trois fois avec une solution de NaCl 150 mmol/l.

L'anticorps marqué à l'iode 125 à tester est dilué de façon que son activité soit égale à $18.10^6$ Bq /50 μl. 50 μl de cette préparation contenant 100 ng par ml d'anticorps sont déposés dans chaque puits. On dépose ensuite 50 μl d'une préparation contenant 10 μg par ml d'un autre anticorps non marqué en solution dans le tampon PBS1. Après une incubation de 3 h à 37°C, les filtres sont lavés cinq fois avec une solution de NaCl 150 mmol/l.

Lorsque les épitopes reconnus par chacun des deux anticorps sont proches, on observe une extinction de la radioactivité mesurée correspondant à l'inhibition de la fixation d'anticorps marqué.

2) Résultats :

Les pourcentages d'inhibition suivants d'un anticorps marqué à l'iode 125 par un anticorps non marqué ont été obtenus :

| Ac Marqué | Ac non marqué | | | | | | | | | |
|---|---|---|---|---|---|---|---|---|---|---|
| | 2E3 1F2 | 10F1 2D4 | 10G9 2D2 | 15C2 2F2 | 16G5 2E7 | 16G5 3D10 | 16F11 2A7 | 16F11 2F10 | 20B2 1B12 | 20B2 3A8 |
| 2E3–1F2 | – | – | – | – | – | – | – | – | – | – |
| 10F1–2D4 | 18 | 63 | 21 | 10 | 39 | 39 | 6 | 17 | 0 | ND |
| 10G9–2D2 | – | – | – | – | – | – | – | – | – | – |
| 15C2–2F2 | 12 | 5 | 0 | 80 | 12 | 8 | 0 | 9 | 0 | ND |
| 16G5–2E7 | 20 | 7 | 4 | 20 | 81 | 64 | 13 | 20 | 1 | ND |
| 16G5–3D10 | 9 | 3 | 0 | 16 | 80 | 66 | 14 | 7 | 5 | ND |
| 16F11–2A7 | – | – | – | – | – | – | – | – | – | – |
| 16F11–2F10 | – | – | – | – | – | – | – | – | – | – |
| 20B2–1B12 | 9 | 3 | 0 | 5 | 5 | 3 | 5 | 1 | 81 | 81 |
| 20B2–3A8 | ND | ND | ND | ND | ND | ND | ND | ND | 89 | 88 |

ND : Non déterminé

– : Anticorps non immunologiquement actif

Ces résultats permettent de déterminer l'existence d'au moins quatre épitopes indépendants sur Bonamia ostreae.

Ces épitopes sont reconnus spécifiquement par les anticorps sélectionnés de la façon suivante :

| EPITOPE | ANTICORPS |
|---|---|
| I | 2OB2-1B12 et 2OB2-3A8 |
| II | 15C2-2F2 |
| III | 16G5-2E7 et 16G5-3D10 |
| IV | 1OF1-2D4 |

L'épitope IV est partiellement recouvert par l'épitope III.

4.2. Détermination des classes et sous-classes

Les classes auxquelles appartiennent les onze anticorps produits ont pu être déterminées. Ils appartiennent à la classe des IgG et plus précisément pour cinq d'entre eux à la sous-classe des IgG1 pour quatre autres à la sous-classe des IgG2a et pour un autre à la sous-classe des IgG3.

| ANTICORPS | SOUS-CLASSE |
|---|---|
| 2E3-1F2 | IgG1 |
| 1OF1-2D4 | IgG1 |
| 1OG9-2D2 | IgG1 |
| 15C2-2F2 | IgG2a |
| 16G5-2E7 | IgG2a |
| 16G5-3D10 | IgG2a |
| 16F11-2A7 | IgG1 |
| 16F11-2F10 | IgG1 |
| 2OB2-1B12 | IgG2a |
| 2OB2-3A8 | IgG3 |

4.3. Détermination des points isoélectriques

Les points isoélectriques de huit anticorps ont été déterminés en mettant en oeuvre la technique d'isofocalisation.

| ANTICORPS | POINT ISOELECTRIQUE |
|---|---|
| 2E3-1F2 | 6,7 à 7,2 |
| 1OF1-2D4 | 6,5 à 7,0 |
| 1OG9-2D2 | 7,5 à 8,0 |
| 15C2-2F2 | 8,2 à 8,5 |
| 16G5-2E7 | 6,3 à 6,6 |
| 16F11-2A7 | 7,0 à 7,3 |
| 16F11-2F10 | 7,0 à 7,3 |
| 2OB2-1B12 | 6,9 à 7,3 |

5 - UTILISATION DES ANTICORPS MONOCLONAUX ANTI-BONAMIA OSTREAE

5.1. Préparation des échantillons à tester

1) Choix du tissu

Bonamia ostreae parasite les cellules sanguines ou hémocytes de l'huître. Le système circulatoire de cet animal n'étant pas clos, les hémocytes se trouvent dispersés dans tous les organes.
Une étude histologique réalisée sur les différents tissus (tissus branchial, palpial, digestif et sanguin (hémolymphe)) montre que l'hémolymphe constitue un tissu de choix en raison, d'une part, de sa représentativité de l'infestation et, d'autre part, de sa facilité de prélèvement par ponction directe dans la cavité péricardique.

2) Traitement de l'hémolymphe

L'hémolymphe après prélèvement est congelée.
Au moment de la réalisation de l'essai elle est décongelée. Ce traitement favorise l'éclatement des cellules sanguines et la libération des protozoaires qu'elles contiennent, ces derniers devenant ainsi accessibles en plus grand nombre aux molécules de l'anticorps monoclonal.

5.2. Préparation des anticorps monoclonaux

Les anticorps utilisés ci-après ont été purifiés par chromatographie d'affinité sur protéine A insolubilisée. Ils ont été marqués à l'aide d'un isotope radioactif selon une procédure habituelle ou d'une enzyme selon un protocole ci-après explicité.

5.3. Essais

1) Visualisation des protozoaires sur un frottis de coeur d'huître par une technique d'immunofluorescence indirecte

L'hémolymphe d'une huître est déposée sur une lame de verre par apposition du coeur. Cette préparation est séchée à l'air et fixée à l'acétone pendant 10 minutes. Elle est alors recouverte d'une préparation de l'anticorps monoclonal, en solution, à raison de 50 $\mu$g par ml, dans du tampon IF. Après 15 minutes d'incubation à température ambiante en chambre humide, la lame est lavée avec du tampon IF puis recouverte d'une solution de sérum de chèvre anti-immunoglobulines de souris conjugué à de l'isothiocyanate de fluorescéine (commercialisé par la Société DIAGNOSTICS PASTEUR (FRANCE) dilué au 1/100 dans du tampon IF-EVANS. Elle est ensuite incubée pendant 30 minutes à température ambiante en chambre humide, puis lavée avec du tampon IF. Une lamelle de verre est déposée sur le frottis en interposant entre lame et lamelle un tampon glycériné commercialisé par la Société DIAGNOSTICS PASTEUR. La lame est examinée à l'aide d'un microscope à épifluorescence en utilisant de l'huile à immersion. Une réaction positive se traduit par une fluorescence verte brillante sur de petites cellules. Celles-ci sont des cellules de Bonamia ostreae. Deux témoins négatifs sont réalisée l'un en utilisant des immunoglobulines de souris non spécifiques en présence d'huître parasitées et l'autre en mettant l'anticorps monoclonal en présence d'huîtres saines. Cet essai a été réalisé à l'aide de chacun des 10 anticorps monoclonaux obtenu à l'exemple 3. Des résultats identiques ont été constatés.

2. Dosage direct sur nitrocellulose avec un anticorps marqué à l'iode 125

Ce dosage consiste à réaliser la fixation immunologique d'un anticorps anti-Bonamia ostreae, marqué à l'iode 125, sur des cellules sanguines d'huître préalablement adsorbées directement sur un filtre de nitrocellulose.
Un filtre de nitrocellulose de 6 mm de diamètre est introduit dans chacun des puits d'une plaque de microtitration. 25 $\mu$l d'hémolymphe prélevée sur une huître sont déposés sur chaque filtre. Après une incubation de 15 heures à 37°C, chaque filtre est saturé pendant 3 heures à 37°C avec 200 $\mu$l de tampon TBS1. Il est ensuite lavé trois fois avec une solution de NaCl 100 mmol/l. 100 $\mu$l d'une préparation de l'anticorps 20B2-1B12 marqué à l'iode 125 dont l'activité a été amenée à 12.10$^6$ Bq /100 $\mu$l par dilution dans du tampon TBS2 sont ajoutés. Après une incubation de 2 heures et 30 minutes à 37°C, le filtre est lavé cinq fois avec une solution de NaCl 100 mmol/l et la radioactivité du filtre est mesurée.

Dans ces conditions, l'hémolymphe d'une huître fortement parasitée permet l'obtention d'un signal de $6.10^6$ Bq , alors que l'hémolymphe d'une huître exempte de Bonamia ostreae permet d'obtenir un signal de $36.10^3$ Bq

### 3. Dosage de type sandwich avec un marquage radioactif

Ce dosage consiste à capturer immunologiquement les parasites présents dans des cellules sanguines d'huître par un anticorps anti-Bonamia ostreae préalablement adsorbé sur une phase solide. Le même anticorps anti-Bonamia-ostreae marqué à l'iode 125, est ajouté de manière qu'il se fixe sur le parasite. Ce dosage est théoriquement plus spécifique que le dosage direct car seuls les parasites pris en sandwich entre les deux anticorps restent présents dans les puits de la plaque de microtitration.

a) Dosage en 1 temps

100 $\mu$l d'une préparation de l'anticorps 20B2-1B12, en solution, à raison de 20 $\mu$g par ml, dans du tampon TBS, sont déposés dans chacun des puits d'une plaque de microtitration. Après 15 heures d'incubation à 4°C, on procède à un lavage à l'aide d'une solution de NaCl 100 mmol/l. Les puits sont alors saturés avec 200 $\mu$l de tampon TBS1 pendant 60 minutes à 37°C. La plaque est ensuite lavée trois fois avec une solution de NaCl 100 mmol/l. 10 $\mu$l d'hémolymphe d'une huître et 90 $\mu$l d'une préparation de l'anticorps 20B2-1B12 marqué à l'iode 125 et dont l'activité a été amené à $15.10.^6$ Bq/90 $\mu$l par dilution dans le tampon TBS2 sont ajoutés simultanément dans chaque puits. La plaque est centrifugée à 350 g pendant 15 minutes. Après une incubation de 2 heures et 30 minutes à 37°C, les puits sont lavés cinq fois avec une solution de NaCl 100 mmol/l et leur radioactivité est mesurée après solubilisation des protéines par 100 $\mu$l de NaOH 4 mol/l et extraction avec le système Cell Harvester commercialisé sous les références 7070, 7071 et 7080 par la société SKATRON (E.U.A.).

b) Dosage en 2 temps

100 $\mu$l d'une préparation de l'anticorps 20B2-1B12, en solution, à raison de 20 $\mu$g par ml, dans du tampon TBS, sont déposés dans chacun des puits d'une plaque de microtitration. Après 15 heures d'incubation à 4°C, on procède à un lavage à l'aide d'une solution de NaCl 100 mmol/l. Les puits sont alors saturés avec 200 $\mu$l de tampon TBS1 pendant 1 heure à 37°C. La plaque est ensuite lavée trois fois avec une solution de NaCl 100 mmol/l. Dans chaque puits 50 $\mu$l d'hémolymphe sont déposés en présence de 50 $\mu$l de tampon TBS2. Les plaques sont centrifugées à 350 g pendant 15 minutes puis incubées pendant 3 heures à 37°C. Les puits sont alors lavés 3 fois avec 200 $\mu$l de NaCl 100 mmol/l. 100 $\mu$l d'une préparation de l'anticorps 20B2-1B12 marqué à l'iode 125 dont l'activité a été amenée à $15.10^6$ Bq /100 $\mu$l par dilution dans le tampon TBS2 sont ensuite incubés 1 heure à 37°C. Après cinq lavages avec une solution de NaCl 100 mmol/l, la radioactivité est mesurée (comme décrit ci-dessus).

Dans l'un et l'autre dosage, il est constaté que l'hémolymphe d'une huître fortement parasitée permet d'obtenir un signal de $1,2.10^6$ Bq alors que l'hémolymphe d'une huître exempte de Bonamia ostreae permet d'obtenir un signal de 8100 Bq.

### 4. Dosage direct sur nitrocellulose avec un anticorps marqué à l'aide d'une enzyme

Pour ce dosage l'anticorps est utilisé couplé à une enzyme. Le couplage est réalisé en activant de la phosphatase alcaline commercialisée sous la référence 567 752 par la société BOEHRINGER par du 4-(p-maleimidophényl)butyrate de succinidinyle commercialisé sous la référence 22315 par la Société PIERCE (E.U.A.) et en activant l'anticorps par de l'anhydride (S-acétyl) mercaptosuccinique commercialisé sous la référence 19 732-7 par la société FLUKA (R.F.A.).

Un filtre de nitrocellulose de 6 mm de diamètre est introduit dans chacun des puits d'une plaque de microtitration. 25 $\mu$l d'hémolymphe d'une huître sont déposés sur chaque filtre. Après une incubation de 15 heures à 37°C, chaque filtre est saturé avec 200 $\mu$l de tampon TBS1 pendant 1 heure à 37°C. Il est ensuite lavé trois fois avec une solution de NaCl 100 mmol/l.100 $\mu$l d'une préparation de l'anticorps 20B2-1B12 marqué à la phosphatase alcaline en solution, à raison de 1 $\mu$g par ml, dans le tampon TBS2, sont ajoutés. Après une incubation d'une heure à 37°C, le filtre est lavé cinq fois avec une solution de NaCl 100 mmol/l. Puis 100 $\mu$l d'une solution de phosphate de paranitrophényl à 1 mg par ml dans un tampon de diéthanolamine 100 mmol/l (pH 9,8) sont ajoutés. Après une incubation de 30 minutes à 20°C, on ajoute enfin dans chaque puits 50 $\mu$l d'une solution de NaOH 1 mol/l. La réaction colorée est mesurée à 405 nm à l'aide d'un spectrophotomètre lecteur de plaques après élimination des filtres de nitrocellulose.

Dans ces conditions il est obtenu une densité optique mesurée à 405 nm de 1,6 à partir d'une hémolymphe d'huître moyennement parasitée, et de 0,16 à partir d'une hémolymphe d'huître exempte de Bonamia

EP 0 288 388 B1

<u>ostreae</u>.

On peut utiliser à la place du phosphate de paranitrophényl un substrat précipitant tel que le phosphate de 5-bromo-4-chloro-3- indolyl commercialisé sous la référence B 8503 par la Société SIGMA (E.U.A.) Dans ces conditions une coloration bleu foncé est obtenue avec de l'hémolymphe d'huître parasitée.

**Revendications**

1. Procédé de détection d'un protozoaire du genre <u>Bonamia</u> parasitant les cellules sanguines d'un mollusque, caractérisé en ce que l'on prélève un échantillon d'hémolymphe d'un mollusque parasité par ledit protozoaire, ledit échantillon étant ensuite soumis à un traitement favorisant l'éclatement des cellules sanguines, et en ce qu'on détecte la présence dudit protozoaire par une technique immunologique à l'aide d'anticorps monoclonaux reconnaissant spécifiquement ledit protozoaire.

2. Procédé selon la revendication 1, caractérisé en ce que le protozoaire appartient à l'espèce <u>Bonamia</u> <u>ostreae</u>.

3. Procédé selon l'une des revendications 1 ou 2, caractérisé en ce que la technique immunologique mise en oeuvre permet, outre la mise en évidence au protozoaire, sa quantification.

4. Procédé selon l'une quelconque des revendications 1 à 3, caractérisé en ce que l'anticorps monoclonal est l'anticorps produit par l'hybridome déposé à l'ECACC sous la référence 87042307.

5. Procédé de préparation d'anticorps monoclonaux reconnaissant spécifiquement un protozoaire du genre <u>Bonamia</u>, caractérisé en ce qù'il comprend :
   - l'extraction dudit protozoaire à partir de l'hémolymphe d'un mollusque parasité par broyage du corps du mollusque duquel on a ôté tout organe de nature fibreuse en présence d'un liquide d'homogénéisation à base d'eau de mer additionnée de 0,5 à 2% (v/v) d'un tensioactif, clarification du broyat et concentration des éléments particulaires dudit broyat, séparation des cellules du protozoaire,
   - la préparation d'une suspension purifiée du protozoaire ainsi extrait, et
   - l'utilisation de ladite suspension pour obtenir les anticorps monoclonaux spécifiques dudit protozoaire.

6. Procédé selon la revendication 5, caractérisé en ce que le liquide d'homogénéisation utilisé dans l'étape d'extraction du protozoaire est de l'eau de mer dont la pression osmotique a été ajustée à 1 osmole filtrée sur une membrane dont la porosité n'excède pas 0,22 $\mu$m et additionnée à raison de 0,5 à 2% (v/v) d'un tensioactif neutre.

7. Procédé selon la revendication 6, caractérisé en ce que le tensioactif est ajouté à l'eau de mer à raison de 1% (v/v).

8. Procédé selon l'une des revendications 6 ou 7, caractérisé en ce que le tensioactif est du monooléate de polyoxyéthylènesorbitane 20.

9. Procédé selon l'une quelconque des revendications 5 à 8, caractérisé en ce que la phase de séparation des cellules du protozoaire comporte plusieurs centrifugations différentielles.

10. Procédé selon la revendication 9, caractérisé en ce qu'il comporte successivement une centrifugation en saccharose sans gradient, une centrifugation en gradient de saccharose et une centrifugation en gradient d'un colloïde polydispersé de silice couverte de polyvinylpyrrolidone.

11. Procédé selon la revendication 9, caractérisé en ce que la phase de séparation comporte en outre une centrifugation isopycnique.

12. Procédé selon l'une quelconque des revendications 5 à 11, caractérisé en ce que l'obtention des anticorps monoclonaux comporte les étapes successives suivantes :
    a) immunisation d'animaux de laboratoire à l'aide de la suspension purifiée de protozoaire,

14

b) hybridation lymphocytaire entre les lymphocytes spléniques d'un animal et des cellules myélomateuses,

c) sélection des hybridomes sécrétant un anticorps monoclonal reconnaissant de manière spécifique un antigène dudit protozoaire, et

d) clonage des hybridomes et production des anticorps.

13. Procédé selon la revendication 12, caractérisé en ce que l'anticorps monoclonal est l'anticorps produit par l'hybridome déposé à l'ECACC sous la référence 87042307.

14. Anticorps monoclonal secrété par l'hybridome déposé à l'ECACC sous la référence 87042307.

15. Hybridome déposé à l'ECACC sous la référence 87042307.

16. Kit de diagnostic pour la détection d'un protozoaire du genre Bonamia parasitant un mollusque, caractérisé en ce qu'il comporte un anticorps monoclonal selon la revendication 14.

**Claims**

1. Method for the detection of a protozoon of the genus Bonamia parasitizing the blood cells of a mollusk, characterized in that a hemolymph sample is taken from a mollusk parasitized by said protozoon, said sample being then sujected to a treatment promoting the rupture of the blood cells, and in that the presence of said protozoon is detected by an immunologic technique using monoclonal antibodies which specifically recognize said protozoon.

2. Method according to claim 1, characterized in that the protozoon belongs to the Bonamia ostreae species.

3. Method according to one of claims 1 or 2, characterized in that the immunologic technique used makes it possible not only to detect the protozoon but also to quantify it.

4. Method according to any one of claims 1 to 3, characterized in that the monoclonal antibody is the antibody produced by the hybridoma deposited at the ECACC under the reference 87042307.

5. Method for the preparation of monoclonal antibodies which specifically recognize a protozoon of the genus Bonamia, characterized in that it comprises:
   - the extraction of said protozoon from the hemolymph of a mollusk parasitized by crushing the body of the mollusk, of which any organ of a fibrous nature was removed, in the presence of a homogenization liquid based on sea water to which there is added 0.5 to 2% (v/v) of a surfactant, clarification of the crushed mixture and concentration of the particulate elements of said crushed mixture, separation of the cells of the protozoon,
   - the preparation of a purified suspension of the protozoon thus extracted, and
   - the use of said suspension in order to obtain the specific monoclonal antibodies of said protozoon.

6. Method according to claim 5, characterized in that the homogenization liquid used in the extraction step of the protozoon is sea water whose osmotic pressure was adjusted to 1 osmol, filtered through a membrane whose porosity does not exceed 0.22 μm and to which there is added, at a ratio of 0.5 to 2% (v/v), a neutral surfactant.

7. Method according to claim 6, characterized in that the surfactant is added to the sea water at a ratio of 1% (v/v).

8. Method according to one of claims 6 or 7, characterized in that the surfactant is polyoxyethylene sorbitan 20 monooleate.

9. Method according to any one of claims 5 to 8, characterized in that the separation phase of the cells of the protozoon comprises a plurality of differential centrifugations.

**10.** Method according to claim 9, characterized in that it successively comprises a centrifugation in sucrose without a gradient, a centrifugation in sucrose with a gradient, and a centrifugation with a gradient of a polydisperse silica colloid covered with polyvinylpyrrolidone.

**11.** Method according to claim 9, characterized in that the phase of separation further comprises an isopycnic centrifugation.

**12.** Method according to one of claims 5 to 11, characterized in that obtaining monoclonal antibodies comprises the following successive steps:
   a) immunization of laboratory animals by means of the purified suspension of protozoon,
   b) lymphocyte hybridation between the splenic lymphocytes of an animal and the myelomatous cells,
   c) selection of the hybridomas secreting a monoclonal antibody which specifically recognise an antigen of said protozoon, and
   d) cloning of the hybridomas and production of the antibodies.

**13.** Method according to claim 12, characterized in that the monoclonal antibody is the antibody produced by the hybridoma deposited at the ECACC under the reference 87042307.

**14.** Monoclonal antibody secreted by the hybridoma deposited at the ECACC under the reference 87042307.

**15.** Hybridoma deposited at the ECACC under the reference 87042307.

**16.** Diagnostic kit for the detection of a protozoon of the genus Bonamia parasitizing a mollusk, characterized in that it comprises a monoclonal antibody according to claim 14.

**Patentansprüche**

**1.** Verfahren zur Feststellung von Protozoen der Gattung Bonamia, die als Parasiten Blutzellen eines Mollusken befallen,
   **gekennzeichnet durch**
   - Entnahme einer Probe der Hämolymphe eines durch das Protozoon als Parasiten befallenen Mollusken,
   - anschließende Behandlung der Probe nach einem Verfahren, welches das Aufbrechen der Blutzellen begünstigt, und
   - Feststellung des Vorliegens des Protozoons durch ein immunologisches Verfahren mit Hilfe monoklonaler Antikörper, die das Protozoon spezifisch erkennen.

**2.** Verfahren nach Anspruch 1, dadurch gekennzeichnet, daß das Protozoon der Species Bonamia ostreae angehört.

**3.** Verfahren nach Anspruch 1 oder 2, dadurch gekennzeichnet, daß das angewandte immunologische Verfahren neben der Feststellung des Protozoons auch seine quantitative Bestimmung erlaubt.

**4.** Verfahren nach einem der Ansprüche 1 bis 3, dadurch gekennzeichnet, daß der monoklonale Antikörper der durch das bei der Hinterlegungsstelle ECACC unter der Hinterlegungsnummer 87042307 hinterlegte Hybridom produzierte Antikörper ist.

**5.** Verfahren zur Herstellung monoklonaler Antikörper, die ein Protozoon der Gattung Bonamia spezifisch erkennen,
   **gekennzeichnet durch** folgende Schritte:
   - Gewinnung des Protozoons, ausgehend von der Hämolymphe eines von den Parasiten befallenen Mollusken durch Zerkleinern des Körpers des Mollusken, aus dem sämtliche Organe faseriger Natur entfernt wurden, in einer Homogenisierungsflüssigkeit auf der Basis von Meerwasser, das mit 0,5 bis 2 % (V/V) eines Tensids versetzt ist,
   - Klärung des Zerkleinerungsprodukts und Aufkonzentrieren der teilchenförmigen Bestandteile des Zerkleinerungsprodukts sowie Abtrennung der Zellen des Protozoons,

- Herstellung einer gereinigten Suspension des so gewonnenen Protozoons und
- Verwendung dieser Suspension zur Herstellung der für dieses Protozoons spezifischen monoklonalen Antikörper.

6. Verfahren nach Anspruch 5, dadurch gekennzeichnet, daß die in der Stufe der Gewinnung des Protozoons verwendete Homogenisierungsflüssigkeit Meerwasser ist, dessen osmotischer Druck auf eine Osmolalität von 1 osm eingestellt wurde und das durch eine Membran einer Porosität von $\leq 0{,}22$ $\mu$m filtriert und mit 0,5 bis 2 % (V/V) eines neutralen Tensids versetzt wurde.

7. Verfahren nach Anspruch 6, dadurch gekennzeichnet, daß das Tensid dem Meerwasser in einer Konzentration von 1 % (V/V) zugegeben wird.

8. Verfahren nach Anspruch 6 oder 7, dadurch gekennzeichnet, daß das Tensid Polyoxyethylensorbitan-20-monooleat ist.

9. Verfahren nach einem der Ansprüche 5 bis 8, dadurch gekennzeichnet, daß die Stufe der Abtrennung der Zellen des Protozoons mehrere differentielle Zentrifugierungen umfaßt.

10. Verfahren nach Anspruch 9, dadurch gekennzeichnet, daß nacheinander eine Zentrifugierung in Saccharose ohne Gradient, eine Zentrifugierung im Saccharosegradienten und eine Zentrifugierung in einem Gradienten eines polydispersen Kolloids von mit Polyvinylpyrolidon überzogener Kieselsäure vorgenommen werden.

11. Verfahren nach Anspruch 9, dadurch gekennzeichnet, daß die Stufe der Abtrennung der Protozoenzellen ferner eine isopyknische Zentrifugierung umfaßt.

12. Verfahren nach einem der Ansprüche 5 bis 11, dadurch gekennzeichnet, daß die Gewinnung der monoklonalen Antikörper die nachstehenden, aufeinanderfolgenden Schritte umfaßt:
    a) Immunisierung von Labortieren mit Hilfe der gereinigten Suspension des Protozoons,
    b) Lymphocytenhybridisierung zwischen Milzlymphocyten eines Tieres und Myelomzellen,
    c) Selektion der Hydridome, die einen monoklonalen Antikörper ausscheiden, der in spezifischer Weise ein Antigen des Protozoons erkennt, und
    d) Klonierung der Hybridome und Herstellung der Antikörper.

13. Verfahren nach Anspruch 12, dadurch gekennzeichnet, daß der monoklonale Antikörper der durch das bei der Hinterlegungsstelle ECACC unter der Hinterlegungsnummer 87042307 hinterlegte Hybridom produzierte Antikörper ist.

14. Monoklonale Antikörper, die von dem bei der Hinterlegungsstelle ECACC unter der Hinterlegungsnummer 87042307 hinterlegten Hybridom ausgeschieden sind.

15. Hybridom, das bei der Hinterlegungsstelle ECACC unter der Hinterlegungsnummer 87042307 hinterlegt ist.

16. Diagnosekit zur Feststellung von Protozoen der Gattung Bonamia, die als Parasiten einen Mollusken befallen, dadurch gekennzeichnet, daß es einen monoklonalen Antikörper nach Anspruch 14 enthält.